# EUROPEAN PATENT APPLICATION

(11) **EP 4 603 580 A1**
(43) Date of publication of application: **20.08.2025**
(21) Application number: 24158130.5
(22) Date of filing: 16.02.2024
(51) Int. Cl.: C12N 9/04, C12N 9/02, C12N 9/88, C12P 7/22, C12R 1/01, C12R 1/19, C12R 1/645, C12R 1/80

(54) **METHOD OF BIOTECHNOLOGICAL VANILLIN OR 4-VINYLGUAIACOL PRODUCTION**

(71) Applicant: Phytowelt Green Technologies GmbH, 41334 Nettetal (DE)
(72) Inventor: TORRES MONROY, Ingrid, 40223 Düsseldorf (DE); BATT, Clemens, 50829 Köln (DE); HEIN, Corinna, 50129 Bergheim (DE); LAUER, Ira, 50829 Köln (DE); SHEREMETIEV, Maria, 50765 Köln (DE); WELTERS, Peter, 41334 Nettetal (DE)
(74) Representative: Grünecker Patent- und Rechtsanwälte PartG mbB

(57) **Abstract**

The invention relates to biotechnological methods for producing highly pure vanillin and 4-vinylguaiacol, as well as microorganisms cultivated in said methods. The microorganisms according to the present invention comprise heterologous nucleotide sequences encoding for the enzymes that catalyse the bioconversion of ferulic acid into 4-vinylguaiacol and vanillin and further comprise genetic modifications to avoid the subsequent reaction of vanillin to vanillyl alcohol, thereby allowing the production of vanillin and 4-vinylguaiacol with improved efficiency and purity.

## Description

### TECHNICAL AREA OF THE INVENTION

The present invention concerns a method for producing highly pure vanillin. The invention further relates to a method for producing highly pure 4-vinylguaiacol. Further, the invention concerns a microorganism cultivated in a method of producing highly pure vanillin and 4-vinylguaiacol. This microorganism comprises heterologous nucleotide sequences encoding the enzymes ferulic acid decarboxylase (FADase) and at least one of aromatic dioxygenase (TtAdo) and aromatic dioxygenase (TtAdo2). Additionally, the present invention relates to the use of a microorganism for the efficient production of highly pure vanillin and 4-vinylguaiacol.

### BACKGROUND OF THE INVENTION

Vanillin (4-hydroxy-3-methoxybenzaldehyde) is a valuable aromatic compound used extensively as a flavouring agent and fragrance in the nutraceutical, cosmetic, and pharmaceutical industries (Havkin-Frenkel & Belanger 2008). The market demand for vanillin has been steadily increasing with a worldwide demand at present of about 20,000 tonnes per year, of which only a minor portion is produced by the extraction from natural vanilla pods.

The extraction of vanillin from vanilla pods is a time-consuming process that requires the cultivation and harvesting of vanilla plants (*Vanilla planifolia*)*.* In addition, the extraction process requires large amounts of solvents, such as ethanol, and the vanillin yield is extremely low, resulting in a high price for natural vanillin, thereby limiting its industrial use (Pérez-Rodriguez et al. 2015). Because of its high costs and low production, natural vanillin extracted from vanilla pods cannot meet the market demand (Ma et al. 2022).

For this reason, about 95% of vanillin is presently produced by chemical synthesis from substances such as petrochemical guaiacol, eugenol, or lignin (Havkin-Frenkel & Belanger 2008; Fache et al. 2015). These methods often involve complex processes that are cost-intensive and have a significant negative environmental impact. Further, the chemical synthesis of vanillin from petrochemical guaiacol requires the usage of toxic reagents and can lead to the production of harmful by-products. Therefore, the use of chemically synthesized vanillin in foods and pharmaceuticals is strictly regulated by US and European legislation (Serra et al. 2005).

4-vinylguaiacol (3-methoxy-4-hydroxystyrene) is another valuable aromatic compound, which is widely used in the food, cosmetic and pharmaceutical industries (Sun et al. 2018, Lui et al. 2023). As for vanillin, the global demand for 4-vinylguaiacol has been increasing in the recent years. 4-vinylguaiacol can be obtained by chemical decarboxylation of ferulic acid, a highly abundant hydroxycinnamate occurring in lignocellulosic materials, such as the cell wall material of plants. The chemical decarboxylation of ferulic acid to 4-vinylguaiacol requires the usage of a metal catalyst combined with high temperatures, which has a significant negative environmental impact (Tinikul et al. 2017; Lui et al. 2023).

Consequently, there is a need for sustainable and efficient methods for the production of natural 4-vinylguaiacol and vanillin that can meet the increasing demand while minimizing the negative environmental impact.

In recent years, the focus has shifted to the biotechnological production of natural vanillin (see Lui et al. 2023; Ma et al. 2022; Banerjee & Chattopadhyay 2018). These biotechnological methods are bioconversions from natural precursors using enzymes or fast-growing organisms such as bacteria or fungi in which the genes required for vanillin production are expressed endogenously or heterologously.

These methods provide low-cost natural vanillin with high efficiency and specificity, low environmental impact, and meet regulatory requirements for food safety (Banerjee & Chattopadhyay 2018). However, the presently known methods for producing vanillin by using naturally vanillin-producing microorganisms or metabolically engineered microorganisms provide low efficiencies and are associated with the formation of undesired aromatic by-products, which may considerably affect the aroma of the product, i.e. vanillin and/or 4-vinylguaiacol. Further, upscaling of these processes is challenging, rendering them unsuitable for large-scale industrial productions.

Microorganisms that are natural vanillin producers, such as *Pseudomonas fluorescens* BF13-1p4 (Di Gioia et al. 2011), *Streptomyces* sp. V-1 (Hua et al. 2007) or *Amycolatopsis* sp. ATCC 39.116 (Valerio et al. 2021; Ma & Daugulis 2014; Fleige et al. 2016), among others, have been used for the production of natural vanillin through biotransformation of ferulic acid. Some of these microorganisms produced large amounts of vanillin, for example, *Amycolatopsis* sp. ATCC 39,116 has been reported to produce 22.3 g/l vanillin (Fleige et al. 2016). However, the use of these microorganisms for large-scale vanillin production is associated with several limitations: increasing the substrate concentrations lowers the conversion rate, and the presence of vanillin-degrading enzymes reduces the concentration of the final product (Lui et al. 2023).

To overcome these limitations, several groups have attempted to develop microorganisms for the biotechnological production of natural vanillin.

Several enzymatic pathways that are generally suitable for the production of vanillin have been reported. For example, *de novo* synthesis of vanillin from glucose can occur using 3-dehydroshikimic acid from the shikimate pathway as an intermediate, which is converted to protocatechuic acid by the enzyme 3-dehydroshikimate dehydratase (3DSD). The subsequent reactions convert protocatechuic acid into protocatechuic aldehyde or vanillic acid by an aromatic carboxylic acid reductase (ACAR) or an O-methyltransferase (OMT), respectively. Finally, vanillin is generated from protocatechuic aldehyde by the OMT and from vanillic acid by the ACAR (Hansen et al. 2009).

The production of vanillin by biotransformation of isoeugenol, eugenol, or ferulic acid has also been reported. Isoeugenol can be directly converted to vanillin using the enzyme isoeugenol monooxygenase (IEM) (Zhao et al. 2018; Wang et al. 2021). Conversely, the conversion of eugenol involves several steps and uses ferulic acid as an intermediate. Eugenol is first converted to coniferyl alcohol by eugenol hydroxylase (EhyAB), which is then converted to coniferyl alcohol by coniferyl alcohol dehydrogenase (CalA), which in turn is converted to ferulic acid by coniferyl aldehyde dehydrogenase (CaIB) (Overhage et al.1999). The resulting ferulic acid can be converted to vanillin via a coenzyme-dependent or a coenzyme-independent pathway (Liu et al. 2023).

In the coenzyme-dependent pathway, feruloyl-CoA synthetase (FCS) converts ferulic acid to feruloyl-CoA, which is then converted to vanillin by an enoyl-CoA-hydratase/aldolase (ECH) (Overhage et al. 1999). The efficiency of this pathway is limited by the intercellular availability of CoA, which is required as a cofactor for the process.

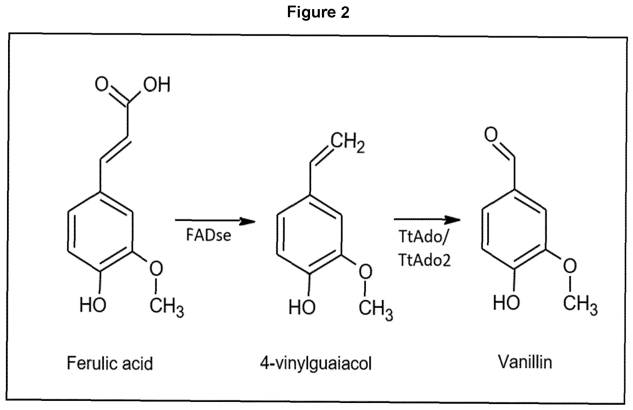

Lee et al. 2009, Biotechnology and Bioengineering, 101(1):200-208*,* reported the production of vanillin by using *E. coli* strains carrying the genes for FCS, ECH, and a citrate synthase (*gltA*) on a plasmid. *Lee et al.* (2009) were able to improve the coenzyme-dependent production of vanillin by the inactivation of a gene encoding for an isocitrate dehydrogenase of the TCA cycle (*icdA*)*,* thereby increasing the intracellular amount of CoA. To date, to the best of the inventors' knowledge, this work reported the highest concentration of vanillin (5.14 g/l) achieved by the biotransformation of ferulic acid in *E. coli.* The maximum efficiency of the method of *Lee et al.* for converting ferulic acid to vanillin was 80% in terms of the molar amount of ferulic acid converted to vanillin (Lee et al. 2009, Figure 2).

In the coenzyme-independent pathway, 4-vinylguaiacol is produced from ferulic acid in a reaction catalysed by a ferulic acid carboxylase (*Gu et al.* 2011), and then vanillin is produced from 4-vinylguaiacol by the action of an aromatic dioxygenase (*Ni et al.* 2018; *Zhao et al.* 2021) or a carotenoid cleavage oxygenase (Cso2) (*Furuya et al.* 2014).

Furuya et al. 2014, ChemBioChem, 15(15):2248-2254, reported the usage of ferulic acid decarboxylase from *Bacillus pumilus* ATCC 14884 and the carotenoid cleavage oxygenase Cso2 from *Caulobacter segnis* ATCC 21756 for the conversion of ferulic acid to vanillin. The heterologous expression of natively folded Cso2 is *E. coli* required the co-expression of additional chaperons. Further, the method of *Furuya et al.* converted ferulic acid to vanillin with an efficiency, calculated from the molar amount of ferulic acid converted to vanillin, of only about 80% after 32 hours.

Zhao et al. 2021, Green Chemistry, 23:838-847, used the phenolic acid decarboxylase from *Bacillus licheniformis* CGMCC7172 and the aromatic dioxygenase from *Thermothelomyces thermophila* or *Thielavia terrestris* for the coenzyme-independent conversion of ferulic acid to vanillin via 4-vinylguaiacol in *E. coli. Zhao et al.* reported a maximum efficiency at pH 7.0 and 37 °C of 16% and 32% by using the aromatic dioxygenases from *Thermothelomyces thermophila* and *Thielavia terrestris,* respectively, with still a high portion of remaining 4-vinylguaiacol, emphasising the low efficiency of the method of *Zhao et al.*

In Ni et al. 2018, J. A. Chem. Soc., 140(47):16001-16005*,* vanillin was produced from ferulic acid by using phenolic acid decarboxylase from *Bacillus coagulans* DSM1 and the aromatic dioxygenase from *Thermothelomyces thermophila.* To reduce the activity of endogenous alcohol dehydrogenases, which catalyse the undesired conversion of vanillin to vanillyl-alcohol, *Ni et al.* performed the conversion at a pH value of 9.5 and a temperature of 50 °C. The method of *Ni et al.* achieved an efficiency of 99.3% (percentage of the molar amount of vanillin produced from ferulic acid) with residual 4-vinylguaiacol present.

The *E. coli* strains used in the methods of the prior art carried the genes encoding for the enzymes for the production of vanillin and 4-vinylguaiacol on plasmids, making it difficult to use this strain in large-scale, industrial bioconversion processes. Further, the presence of these genes on plasmids requires a continuous presence of antibiotics in the growth medium to avoid the loss of genetic information by plasmid curing.

Additionally, endogenous alcohol dehydrogenases in microorganisms, including *E. coli,* convert vanillin into vanillyl alcohol, which reduces the efficiency of the process by reducing the yield of vanillin and leads to the formation of aromatic by-products.

In the fragrance and flavour industry, high purity of an odorant or aroma is of central importance. Some odorous substances are already perceptible at a very low odour threshold of about 0.01 pg/I air (see Armanino et al. 2020, Angew Chem Int Ed, 59(38):16310-16344)*.* The odour threshold for pure vanillin is 0.02 ng/l air. 4-alkylguaiacol derivatives, including 4-vinylguaiacol, possess spicy or smoky tonality and thus differ from the odour of vanillin. 4-Vinylguaiacol, which is the most frequently occurring by-product in the biotechnological production of vanillin from ferulic acid, is associated with the sensory descriptors "spices" and "cloves", and the threshold value for taste perception in water is 2.7 µg/l (see Knapp et al. 2010, Mitteilungen Klosterneuburg, 60:268-277)*.* With increasing concentrations, the olfactory impression of 4-vinylguaiacol is described as "chemical-medicinal", "bacon", "rubber", "hay", and "barn". Given the low perception threshold of some of the by-products that can be formed during the biotechnological production of vanillin, the production of pure vanillin is of paramount importance.

As described above, vanillin and 4-vinylguiaiacol are important aromatic compounds used in a multitude of industrial applications with increasing demand. While the isolation of natural vanillin from vanilla pods is highly cost-intensive and limited in production, the presently available methods for the chemical synthesis of vanillin or 4-vinylguaiacol can lead to the generation of by-products and has a high environmental impact. Further, the biotechnological production of vanillin by the presently available methods is associated with only moderate efficiency and the formation of aromatic by-products, which influence the taste and flavour of the composition, and the methods are not suitable for upscaling the production process.

The problem addressed by the present invention is the cost-effective, efficient production of highly pure vanillin and/or 4-vinylguaiacol by means of an environment friendly and sustainable method in high quantity and purity.

### SUMMARY OF THE INVENTION

The above-formulated problem is solved by the provision of a method of producing vanillin comprising culturing a microorganism that comprises heterologous nucleotide sequences integrated in the genome that encode a ferulic acid decarboxylase (FADase) and at least one of a first aromatic dioxygenase and a second aromatic dioxygenase.

The above-formulated problem is also solved by the provision of a method of producing 4-vinylguaiacol comprising culturing a microorganism that comprises a heterologous nucleotide sequence integrated in the genome that encodes ferulic acid decarboxylase (FADase).

Further, the microorganism provided by the invention contributes to the solution of the problem, wherein the microorganism comprises heterologous nucleotide sequences integrated in the genome that encode a ferulic acid decarboxylase (FADase), and at least one of a first aromatic dioxygenase and a second aromatic dioxygenase or wherein the microorganism comprises a heterologous nucleotide sequence integrated into the genome that encodes ferulic acid decarboxylase (FADase).

Finally, the use of the microorganism also contributes to the solution of the problem, wherein the microorganism comprises heterologous nucleotide sequences integrated in the genome that encode a ferulic acid decarboxylase (FADase), and at least one of a first aromatic dioxygenase and a second aromatic dioxygenase or wherein the microorganism comprises a heterologous nucleotide sequence integrated into the genome that encodes ferulic acid decarboxylase (FADase).

The method, microorganisms, and use of the microorganisms according to the present invention enable the efficient bioconversion of ferulic acid into vanillin with clearly improved efficiency and purity compared to the state of the art, as well as the efficient bioconversion of ferulic acid into 4-vinylguaiacol, with clearly improved efficiency and purity compared to the state of the art.

The present invention is, among other things, characterized by an improved efficiency of the conversion of ferulic acid into 4-vinylguaiacol and vanillin compared to the state of the art. The present invention enables the conversion of ferulic acid into vanillin or 4-vinylguaiacol with an efficiency of 100%.

Further, the present invention enables the production of 100% pure vanillin or 100% pure 4-vinylguaiacol.

These aspects considerably contribute to a cost effective production of vanillin and 4-vinylguaiacol with high efficiency and purity.

Further, the methods of the present invention do not require cost-intensive chemical synthesis steps or toxic reagents. Thus, the methods of the present invention are more environment friendly and more sustainable than the traditional chemical synthesis of vanillin or 4-vinylguaiacol of the state of the art.

### BRIEF DESCRIPTION OF THE FIGURES

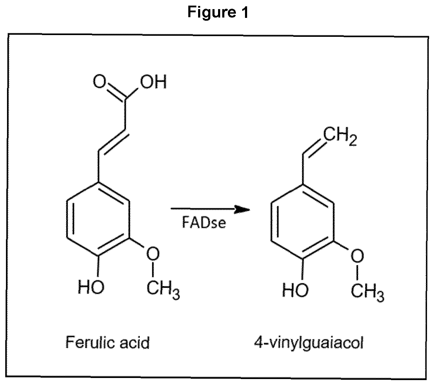

**Figure 1****:** 4-vinylguaiacol synthesis pathway from ferulic acid. Pathway of the bioconversion of ferulic acid into 4-vinylguaiacol catalysed by ferulic acid decarboxylase (FADase) according to the present invention. FADase: ferulic acid decarboxylase from *Enterobacter so.* Px6-4.
**Figure 2****:** Vanillin synthesis pathway from ferulic acid. Pathway of the coenzyme-independent bioconversion of ferulic acid into vanillin via 4-vinylguaiacol according to the present invention. Ferulic acid decarboxylase (FADase) catalyses the conversion of ferulic acid to 4-vinylguaiacol. Then, aromatic dioxygenase (for example TtAdo or TtAdo2) catalyses the conversion of 4-vinylguaiacol to vanillin. FADase: ferulic acid decarboxylase from *Enterobacter* so. Px6-4, TtAdo: aromatic dioxygenase from *Myceliophthora thermophila,* TtAdo2: aromatic dioxygenase from *Thermothielavioides terrestris.*

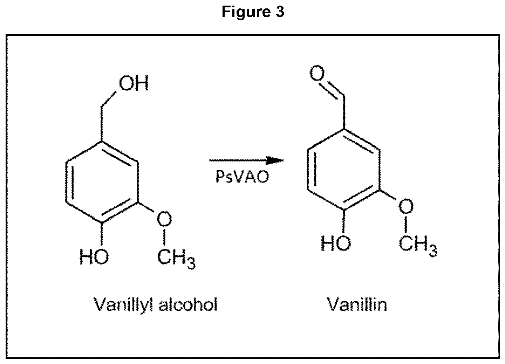

**Figure 3****:** Conversion of vanillyl alcohol into vanillin. Pathway of the bioconversion of vanillyl alcohol into vanillin catalysed by vanillyl alcohol oxidase from *Penicillium simplicissimum* (PsVAO).

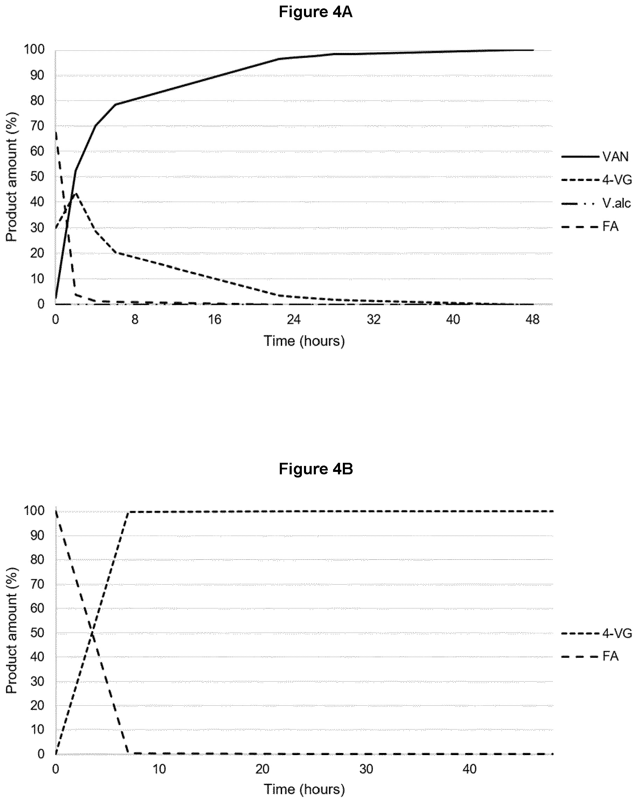

**Figure 4A****:** Time course of the vanillin and 4-vinylguaiacol production. Product amount of vanillin (VAN), 4-vinylguaiacol (4-VG), and vanillyl alcohol (V.alc) produced from ferulic acid (FA) at different time points by using a method according to the present invention. An *E. coli* strain according to the present invention was used as a biocatalyst comprising heterologous nucleotide sequences encoding for FADase, TtAdo, TtAdo2, and PsVAO stably integrated into the genome. The nucleotide sequence encoding for FADase was integrated into the gene *yahK* and the nucleotide sequence encoding for TtAdo was integrated into the *yqhC-dkgA* operon in order to disrupt the activity of these endogenous alcohol dehydrogenases. The bioconversion was performed in 750 ml Riesenberg media, supplemented with 15 g/l glucose, inoculated with a starting OD600 of 0.2, and incubated at 28 °C at a pH value of 7 for 22 hours. After the culture had reached an OD600 of 20, 5mM ferulic acid was added (time 0 in the graph), the temperature was set to 42 °C, and the pH value was adjusted to 8. The bioconversion was performed for 48 hours. Samples were taken and extracted with methanol and the extracted products were analysed by HPLC. The product amount is depicted as a percentage in relation to the amount of substance of ferulic acid used as the starting material for the bioconversion.
**Figure 4B****:** Time course of the 4-vinylguaiacol production. Product amount of 4-vinylguaiacol (4-VG) produced from ferulic acid (FA) at different time points by using a method according to the present invention. An *E. coli* strain according to the present invention was used as a biocatalyst comprising a heterologous nucleotide sequence encoding for FADase, stably integrated into the gene *yahK,* leading to its inactivation. The bioconversion was performed in 750 ml Riesenberg media, supplemented with 15 g/l glucose, inoculated with a starting OD600 of 0.2, and incubated at 28 °C at pH 7 for 17h. After the culture had reached an OD600 of 30, 5 mM ferulic acid was added (time 0 in the graph). The bioconversion was performed for 48 hours. Samples were taken and extracted with methanol and the products were analysed by HPLC. The product amount is depicted as a percentage in relation to the amount of substance of ferulic acid used as the starting material for the bioconversion.

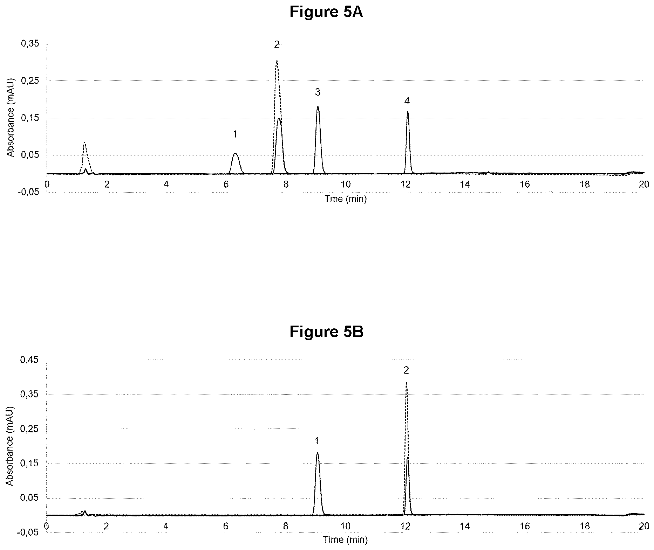

**Figure 5A****:** HPLC chromatogram of the products of bioconversion of ferulic acid to vanillin according to the present invention. Samples of the bioconversion depicted in Figure 4A were extracted with methanol and analysed by HPLC (dashed line). For the correct assignment of the signals, commercially available, highly pure analytical standards of vanillyl alcohol (1), vanillin (2), ferulic acid (3), and 4-vinylguaiacol (4) were used (solid line).
**Figure 5B****:** HPLC chromatogram of the products of bioconversion of ferulic acid to 4-vinylguaiacol according to the present invention. Samples of the bioconversion depicted in Figure 4B were taken and extracted with methanol. The extracted products of the bioconversion were analysed by HPLC (dashed line). For the correct assignment of the signals, commercially available, highly pure analytical standards of ferulic acid (1) and 4-vinylguaiacol (2) were used (solid line).

### DETAILED DESCRIPTION OF THE INVENTION

The present invention is not limited to the specifically mentioned products and methods herein but provides general technical teaching, which enables the skilled person to achieve the advantages described herein. The used terminology should not limit the general technical teaching described herein in any form but serves merely to describe the specific embodiments.

The description of the embodiments has been presented for purposes of illustration only. It is not exhaustive and does not limit the embodiments to the precise form disclosed. While several exemplary embodiments and features are described, modifications, adaptations, and other implementations may be possible, without departing from the spirit and scope of the embodiments. Accordingly, unless explicitly stated otherwise, the descriptions relate to one or more embodiments and should not be construed to limit the embodiments as a whole. This is true regardless of whether or not the disclosure states that a feature is related to "a," "the," "one," "one or more," "some," or "various" embodiments. Also, stating that a feature may exist indicates that the feature may exist in one or more embodiments

Unless defined otherwise, all technical and scientific terms used herein have the same meaning as commonly understood by one of ordinary skill in the art to which the disclosure belongs.

All publications, including but not limited to patents, patent applications, and scientific publications, cited in this description are herein incorporated by reference for all purposes as if each individual publication were specifically and individually indicated to be incorporated by reference.

As used herein, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

Section headings throughout the description are for organizational purposes only. In particular, they are not intended as limiting for various embodiments described therein, and it is to be understood that embodiments (and features therein) described under one subheading may be freely combined with embodiments (and features therein) described under another subheading.

The "accession numbers" used herein (GenBank accession number-GenBank) serve for the unambiguous characterization of nucleotide sequences or amino acid sequences and are taken from the webpage of the NCBI (National Center for Biotechnology Information).

The use of the term "comprising" as well as other grammatical forms such as "comprises" and "comprised" is not limiting. The terms "comprising", "comprises", and "comprised" should be understood as referring to an open-ended description of an embodiment of the present invention that may, but does not have to, include additional technical features in addition to the explicitly stated technical features. In the same sense the term "involving" as well as other respective grammatical forms such as "involves" and "involved" is not limiting. The same applies to the term "including" and other grammatical forms such as "includes" and "included". Section headings throughout the description are for organizational purposes only. In particular, they are not intended as limiting for various embodiments described therein, and it is to be understood that embodiments (and features therein) described under one subheading may be freely combined with embodiments (and features therein) described under another subheading. Further, the terms "comprising", "involving", and "including", and any grammatical forms thereof, are not to be interpreted to exclusively refer to embodiments that include additional features to those explicitly recited. These terms equally refer to embodiments that consist of only those features that are explicitly mentioned.

As used herein, the term "nucleic acid" refers to an oligomer or polymer of naturally occurring or modified nucleotides. The nucleic acid may also comprise nucleotide substitutions relative to a reference sequence. For instance, the nucleic acid may contain 1, 2, 3, 4, 5, 6, 7, 8, 9 or 10 nucleotide substitutions. The nucleic acid may have 100 %, 95%, 90%, 85%, 80%, 75%, 70%, 65%, 60%, 55%, or 50% sequence identity to a reference sequence. A reference sequence may be a nucleic acid with a sequence according to SEQ ID NO: 1 to SEQ ID NO: 8. A nucleic acid may be a ribonucleic acid (RNA) or a deoxyribonucleic acid (DNA) or a hybrid between RNA and DNA. The nucleic acid may be single or double-stranded. The double-stranded nucleic acid may be RNA-RNA, RNA-DNA (hybrid) or DNA-DNA. Double-strand formation occurs through, for example, hydrogen bonds according to the Watson-Crick-base pairing. The nucleic acid may have one or two 3' overhangs. The nucleic acid may have one or two 5' overhangs. The nucleic acid may have one or two blunt ends. Combinations of the above-referenced overhangs or blunt ends are also included.

The nucleic acid can be defined by a sequence of nucleotides indicated in the commonly accepted letter code for the base of the nucleotide, A (adenine), C (cytosine), G (guanine), T (thymine) and U (uracil). "Sequence" as used herein refers to a sequence of nucleotides or a sequence of amino acids.

The term "gene" as used herein specifically refers to nucleic acid molecules or polynucleotides of the disclosure which can be DNA, cDNA, genomic DNA, synthetic DNA, or, RNA, and can be double-stranded or single-stranded, the sense and/or an antisense strand. The term "gene" shall particularly apply to the polynucleotide(s) as used herein, e.g. as full-length nucleotide sequence or fragments or parts thereof, which encodes a polypeptide with enzymatic activity, e.g. an enzyme of a metabolic pathway, or fragments or parts thereof, respectively.

The term "*yahK*" as used herein describes the gene of *Escherichia coli* BL21 alcohol dehydrogenase with the GenBank accession number GenBank: QJZ06966.1.

The term *"yqhC-dkgA* operon" as used herein describes the *Escherichia coli* BL21 alcohol dehydrogenase operon, comprising the nucleotide sequences of *yqhC,* yqhD, and *dkgA,* with the GenBank protein accession numbers GenBank: QJZ09849.1, QJZ09848.1, and QJZ09847.1, respectively.

The term "yield" as used herein describes the amount of a produced material based on a determined culture volume (liquid culture of a microorganism) or the isolated dry matter from a determined culture volume or based on a different reference value. The term "amount" as used herein describes the amount of substance of a material or a different measure, whose value is directly dependent on the amount of substance of the material, for example, the peak area of an HPLC absorption chromatogram.

Within the present disclosure, an efficiency of 100% refers to the conversion of substantially each molecule of ferulic acid into 4-vinylguaiacol or vanillin in the absence of remaining ferulic acid or any other by-product. However, a person skilled in the art is aware that an efficiency of 100% has to be interpreted as "substantially 100%" within the confines of the detection limit. The person skilled in the art is further aware that the detection process of a product is subject to a certain measurement inaccuracy.

The term "purity" as used herein refers to the absence of contaminants or unwanted by-products. A purity of 100% refers to the production of the desired product in the absence of any detectable side products. For example, pure vanillin (100% purity) contains only vanillin in the absence of 4-vinylguaiacol, ferulic acid or vanillyl alcohol.

In the present disclosure, a purity or efficiency of "100%" has to be interpreted as "substantially 100%".

The term "natural vanillin" as used herein describes vanillin derived from natural starting materials, including vanillin extracted from vanilla pods as well as biotechnologically produced vanillin from natural starting material comprising the production of vanillin from ferulic acid according to the process of the present invention. Natural vanillin is distinguished herein from chemically synthesized vanillin.

The term "sequence identity" as used herein describes the agreement of two nucleotide sequences or amino acid sequences, given in percent, and depends on the number of identical positions between the two sequences, wherein the number and length of gaps that need to be introduced to achieve an optimal sequence alignment is considered. As used herein, the sequence identity is determined according to the BLAST algorithm (Altschul et al., 1990). As known to the skilled person, the sequence identity can be determined according to the BLAST algorithm for nucleotide sequences (blastn) or amino acid sequences (blastp) simply on the NCBI webpage (http://blast.ncbi.nlm.nih.gov/Blast.cgi).

The term "heterologous nucleotide sequence" as used herein refers to genetic material that originates from a different species or source than the host organism in which it is introduced. Methods for expressing a heterologous nucleotide sequences in a microorganism are well known to a person skilled in the art.

### Method of Producing 4-Vinylquaiacol

An aspect of the present invention concerns a method for producing 4-vinylguaiacol from ferulic acid (Figure 1).

The method comprises the step of culturing a microorganism that comprises a heterologous nucleotide sequence integrated into the genome that encodes ferulic acid decarboxylase (FADase).

In a preferred embodiment, the ferulic acid decarboxylase is the ferulic acid decarboxylase (FADase) from *Enterobacter sp.* Px6-4 or has sufficient ferulic acid decarboxylase acitivity for the production of 4-vinylguaiacol and has at least 70% or at least 80%, 85%, 90%, 91%, 92%, 93%, 94%, 95%, 96%, 97%, 98%, 99%, or 100% sequence identity with the nucleotide sequence according to SEQ ID NO.1. In a preferred embodiment, the ferulic acid decarboxylase (FADase) has sufficient ferulic acid decarboxylase activity for the production of 4-vinylguaiacol and has at least 80%, 85%, 90%, 91%, 92%, 93%, 94%, 95%, 96%, 97%, 98%, 99%, or 100% sequence identity with the amino acid sequence of ferulic acid decarboxylase from *Enterobacter sp.* Px6-4 with the GenBank accession number GenBank: ACJ26748.1 according to SEQ ID NO.2. In a particularly preferred embodiment, the nucleotide sequence that encodes the ferulic acid decarboxylase (FADase) has at least 90% sequence identity with the sequence according to SEQ ID NO.1. In a particularly preferred embodiment, the amino acid sequence of the ferulic acid decarboxylase (FADase) has at least 90% sequence identity with the amino acid sequence according to SEQ ID NO. 2.

The method enables the production of 4-vinylguaiacol from ferulic acid with a purity of substantially 100%. The high purity of the product is of utmost importance for use in the flavour and fragrance industry. Further, the genomic integration of the nucleotide sequence encoding for FADase enables culturing of said microorganism in the absence of antibiotics and facilitates the upscaling of the process for large-scale industrial production. The method of the present invention for the production of 4-vinylguaiacol by using FADase according to SEQ ID NO.1 and SEQ ID NO.2 provides a surprisingly high efficiency for the conversion of ferulic acid to 4-vinylguaiacol and a high stability over a broad range of temperatures and pH values.

The method of producing 4-vinylguaiacol can be performed with a temperature in the range of 10 °C to 70 °C. Preferably, the method is performed in a range of 25 °C to 40 °C. More preferable are embodiments, wherein the method of producing 4-vinylguaiacol is performed at a temperature of 28 °C. The method of producing 4-vinylguaiacol can be performed within at a pH value between 4 to 12. Preferably, the method of producing 4-vinylguaiacol is performed at a pH value of 6.5 to 8. More preferable are embodiments, wherein the method of producing 4-vinylguaiacol is performed at a pH value of 7. In the most preferred embodiment, the method of producing 4-vinylguaiacol is performed at a temperature of 28 °C at a pH value of 7.

### Method of Producing Vanillin

The present invention also relates to a method for producing vanillin.

The method of producing vanillin according to the present invention comprises culturing a microorganism that comprises heterologous nucleotide sequences integrated in the genome that encode a ferulic acid decarboxylase (FADase), and at least one of a first aromatic dioxygenase and a second aromatic dioxygenase.

The first aromatic dioxygenase and the second aromatic dioxygenase are different from one another concerning their nucleotide sequences and amino acid sequences. Both enzymes catalyse the conversion of 4-vinylguaiacol to vanillin. Surprisingly, the Inventors found that the combination of a first aromatic dioxygenase and a second aromatic dioxygenase catalyses the conversion of 4-vinylguaiacol to vanillin with an outstanding high efficiency. Preferably, the microorganism comprises the heterologous nucleotide sequences integrated in the genome that encode one copy of the gene of FADase, one copy of the gene of a first aromatic dioxygenase, and two copies of the gene of a second aromatic dioxygenase.

The method enables the production of highly pure vanillin from ferulic acid via 4-vinylguaiacol as an intermediate (Figure 2). In a preferred embodiment, the ferulic acid decarboxylase is the ferulic acid decarboxylase (FADase) from *Enterobacter sp.* Px6-4 with the GenBank accession number GenBank: ACJ26748.1 or has at least 70%, or at least 80%, 85%, 90%, 91%, 92%, 93%, 94%, 95%, 96%, 97%, 98%, 99% or 100% sequence identity with the nucleotide sequence of ferulic acid decarboxylase from *Enterobacter sp.* Px6-4 according to SEQ ID NO.1. The ferulic acid decarboxylase (FADase) has sufficient ferulic acid decarboxylase activity for the production of 4-vinylguaiacol, in particular as an intermediate in the process of producing vanillin. In a preferred embodiment, the nucleotide sequence encoding for the ferulic acid decarboxylase (FADase) is codon optimized for improving the heterologous expression in the microorganism used in the method of the present invention. In a preferred embodiment, the ferulic acid decarboxylase (FADase) has at least 80%, 85%, 90%, 91%, 92%, 93%, 94%, 95%, 96%, 97%, 98%, 99% or 100% sequence identity with the amino acid sequence of ferulic acid decarboxylase from *Enterobacter sp.* Px6-4 according to SEQ ID NO.2.

In a preferred embodiment of the method of producing vanillin according to the present invention, which can be combined with any of the preceding and subsequent embodiments, the first aromatic dioxygenase is *Myceliophthora thermophila* aromatic dioxygenase (TtAdo) with the GenBank accession number GenBank: AEO60340.1. In a preferred embodiment, the nucleotide sequence encoding for the first aromatic dioxygenase is codon optimized for improving the heterologous expression in the microorganism used in the method of the present invention. Particularly preferred are embodiments, in which the first aromatic dioxygenase has sufficient aromatic dioxygenase activity for the production of vanillin from 4-vinylguaiacol and has at least 70% or at least 80%, 85%, 90%, 91%, 92%, 93%, 94%, 95%, 96%, 97%, 98%, 99% or 100% sequence identity with the sequence of aromatic dioxygenase (TtAdo) from *Myceliophthora thermophila* according to SEQ ID NO. 3. In a particularly preferred embodiment, the nucleotide sequence that encodes the first aromatic dioxygenase has at least 90% sequence identity with the sequence according to SEQ ID NO.3. In a preferred embodiment, the aromatic dioxygenase (TtAdo) has sufficient aromatic dioxygenase activity for the production of vanillin from 4-vinylguaiacol and has at least 80%, 85%, 90%, 91%, 92%, 93%, 94%, 95%, 96%, 97%, 98%, 99% or 100% sequence identity with the amino acid sequence of aromatic dioxygenase from *Myceliophthora thermophila* according to SEQ ID NO.4. In a particularly preferred embodiment, the amino acid sequence that encodes the first aromatic dioxygenase has at least 90% sequence identity with the sequence according to SEQ ID NO.4.

In a preferred embodiment of the method of producing vanillin according to the present invention, which can be combined with any of the preceding and subsequent embodiments, the second aromatic dioxygenase is *Thermothielavioides terrestris* aromatic dioxygenase (TtAdo2) with the GenBank accession number GenBank: AEO67587.1. In a preferred embodiment, the nucleotide sequence that encodes the second aromatic dioxygenase is codon optimized for improving the heterologous expression in the microorganism used in the method of the present invention. Particularly preferred are embodiments, in which the second aromatic dioxygenase has sufficient aromatic dioxygenase activity for the production of vanillin from 4-vinylguaiacol and has at least 70% or at least 80%, 85%, 90%, 91%, 92%, 93%, 94%, 95%, 96%, 97%, 98%, 99% or 100% sequence identity with the sequence of aromatic dioxygenase (TtAdo2) from *Thermothielavioides terrestris* according to SEQ ID NO. 5. In a particularly preferred embodiment, the nucleotide sequence that encodes the second aromatic dioxygenase has at least 90% sequence identity with the sequence according to SEQ ID NO.5. In a preferred embodiment, the aromatic dioxygenase (TtAdo2) has sufficient aromatic dioxygenase activity for the production of vanillin from 4-vinylguaiacol and has at least 80%, 85%, 90%, 91%, 92%, 93%, 94%, 95%, 96%, 97%, 98%, 99% or 100% sequence identity with the amino acid sequence of aromatic dioxygenase from *Thermothielavioides terrestris* according to SEQ ID NO.6. In a particularly preferred embodiment, the amino acid sequence that encodes the second aromatic dioxygenase has at least 90% sequence identity with the sequence according to SEQ ID NO.6.

In some embodiments, vanillin or 4-vinylguaiacol is extracted by using an organic solvent, preferably methanol. The purity of vanillin produced by the method of the present invention can be substantially 100%. Further, the efficiency of the conversion of ferulic acid to vanillin by using the method of the present invention can be substantially 100%.

Endogenous alcohol dehydrogenases in microorganisms, including *E. coli,* convert vanillin into vanillyl alcohol. Said formation of aromatic by-products reduces the purity of the vanillin and the efficiency of the process for producing vanillin. This problem is solved in embodiments of the present invention by the expression of enzymes that catalyse the reaction of vanillyl alcohol to vanillin and additionally by the inactivation of endogenous alcohol dehydrogenases. In some embodiments, vanillyl alcohol formed as by-product can be converted to vanillin by the activity of a vanillyl alcohol oxidase (Figure 3). In a particularly preferred embodiment of the method of producing vanillin according to the present invention, which can be combined with any of the preceding and subsequent embodiments, the microorganism further comprises a heterologous nucleotide sequence that encodes for a vanillyl alcohol oxidase. In a preferred embodiment, the nucleotide sequence encoding for the vanillyl alcohol oxidase is codon optimized for improving the heterologous expression in the microorganism used in the method of the present invention. Particularly preferred are embodiments, in which the vanillyl alcohol oxidase is the vanillyl alcohol oxidase from *Penicillium simpliocissimum* with the GenBank accession number GenBank: CAA75722.1 or has sufficient vanillyl alcohol oxidase activity for the production of vanillin from vanillyl alcohol and has at least 70% or at least 80%, 85%, 90%, 91%, 92%, 93%, 94%, 95%, 96%, 97%, 98%, 99% or 100% sequence identity with the nucleotide sequence of vanillyl alcohol oxidase (PsVAO) from *Penicillium simplicissimum* according to SEQ ID NO. 7. In a particularly preferred embodiment, the vanillyl alcohol oxidase has at least 90% sequence identity with the nucleotide sequence of vanillyl alcohol oxidase (PsVAO) from *Penicillium simplicissimum* according to SEQ ID NO. 7. In a preferred embodiment, the vanillyl alcohol oxidase has sufficient vanillyl alcohol oxidase activity for the production of vanillin from vanillyl alcohol and has at least 80%, 85%, 90%, 91%, 92%, 93%, 94%, 95%, 96%, 97%, 98%, 99% or 100% sequence identity with the amino acid sequence of vanillyl alcohol oxidase from *Penicillium simplicissimum* according to SEQ ID NO.8. In a particularly preferred embodiment, the vanillyl alcohol oxidase has at least 90% sequence identity with the amino acid sequence of vanillyl alcohol oxidase (PsVAO) from *Penicillium simplicissimum* according to SEQ ID NO. 8.

The method can further comprise the inactivation of endogenous alcohol dehydrogenases to prevent the conversion of vanillin to vanillyl alcohol. The activity of endogenous alcohol dehydrogenases can be reduced by thermal inactivation of endogenous alcohol dehydrogenases. In some embodiments, the method comprises the thermal inactivation of alcohol dehydrogenases by performing the method at a temperature above 37 °C, preferably at 42 °C. In a particularly preferred embodiment of the method of producing vanillin according to the present invention, which can be combined with any of the preceding and subsequent embodiments, the microorganism has a reduced expression of alcohol dehydrogenases. Particularly preferred are embodiments, in which the microorganism has a reduced expression of *yahK, yqhC, yqhD,* and/or *dkgA* compared to the expression of the respective gene in the wild type microorganism. In preferred embodiments, the expression of these genes is reduced compared to the expression of the respective gene in the wild type microorganism by gene inactivation. Methods for gene inactivation are well known to a person skilled in the art of the present invention and include, but are not limited to, the integration of nucleotide sequences into the genes *yahK, yqhC, yqhD,* and/or *dkgA.* Particularly preferred are embodiments, in which the reduced expression of *yahK, yqhC,* yqhD, and/or *dkgA* results from the integration of genetic elements into the genes *yahK, yqhC,* yqhD, and/or *dkgA.* In some embodiments, the genetic elements integrated into the genes *yahK, yqhC,* yqhD, and/or *dkgA* include at least one heterologous nucleotide sequence that encodes one or more of FADase, TtAdo, TtAdo2, and PsVAO. The integration of genetic elements preferably prevents or reduces the expression of said alcohol dehydrogenases. In some embodiments, the activity of the gene products of *yahK, yqhC, yqhD,* and/or *dkgA* is prevented or reduced compared to the activity of the respective gene in the wild type microorganism. In a particularly preferred embodiment, the heterologous nucleotide sequence encoding for FADase is integrated in the gene *yahK,* thereby preventing the expression of *yahk.* In a particularly preferred embodiment, the heterologous nucleotide sequence encoding for TtAdo is integrated in the *yqhC-dkgA* operon, thereby preventing the expression of *yqhC, yqhD,* and *dkgA.* Particularly preferred are embodiments in which the activity of endogenous alcohol dehydrogenases is inhibited by performing the method at an increased temperature, as described herein, preferably at 42 °C, which is sufficiently high to reduce the activity of endogenous alcohol dehydrogenases, and by preventing the expression of *yahK, yqhC, yqhD,* and/or *dkgA* by integrating the heterologous nucleotide sequences encoding for FADase, TtAdo, TtAdo2, and/or PsVAO into the genes *yahK, yqhC, yqhD,* and/or *dkgA.*

The Inventors found that the method of producing vanillin and 4-vinylguaiacol leads to an outstanding high efficiency if performed under conditions, which enable the proliferation of the microorganism. Preferably, the method is performed at temperatures that are sufficiently high to inhibit the activity of endogenous alcohol dehydrogenases but still enable the proliferation of the microorganism. Particularly preferred are embodiments in which the method is performed at a temperature range between 37-45 °C. Most preferred is a temperature of 42 °C.

In some embodiments of the method of producing vanillin according to the present invention, the microorganism comprises the heterologous nucleotide sequence integrated in the genome that encodes a FADase and a first aromatic dioxygenase; the microorganism comprises the heterologous nucleotide sequence integrated in the genome that encodes a FADase and a second aromatic dioxygenase; or the microorganism comprises the heterologous nucleotide sequences integrated in the genome that encode a FADase, a first aromatic dioxygenase, and a second aromatic dioxygenase. It is preferred that the microorganism comprises the heterologous nucleotide sequences integrated in the genome that encode FADase, a first aromatic dioxygenase, and a second aromatic dioxygenase.

In the most preferred embodiment, the method of producing vanillin according to the present invention comprises culturing a microorganism that comprises heterologous nucleotide sequences that encode a ferulic acid decarboxylase (FADase), a first aromatic dioxygenase (TtAdo) and two copies of a second aromatic dioxygenase (TtAdo2), wherein the microorganism further comprises a heterologous nucleotide sequence that encodes for vanillyl alcohol oxidase (PsVAO), wherein the microorganism is *Escherichia coli* which has a reduced expression of *yahK, yqhC, yqhD,* and/or *dkgA* compared to the expression of the respective gene in the wild type *E. coli* resulting from the integration of the heterologous nucleotide sequences encoding for FADase into the gene *yahK* and the integration of the heterologous nucleotide sequence encoding for TtAdo into the *yqhC-dkgA* operon and wherein the method is performed at a 42°C to reduce the activity of endogenous alcohol dehydrogenases and to enable the proliferation of the microorganism.

In a particularly preferred embodiment, which can be combined with any of the preceding and subsequent embodiments, the heterologous nucleotide sequence that encode vanillyl alcohol oxidase is integrated into the genome. Particularly preferred are embodiments, in which the heterologous nucleotide sequences that encode one or more of FADase, a first aromatic dioxygenase, a second aromatic dioxygenase, and a vanillyl alcohol oxidase are integrated into the genome. The heterologous nucleotide sequences can be integrated into the genome by using methods that are known to a skilled person in the art of the present invention. Particularly preferred are embodiments, in which the heterologous nucleotide sequences are integrated into the genome of a microorganism by homologous recombination. The genomic integration of these nucleotide sequences facilitates the upscaling of the method for use in large-scale industrial bioconversion processes and prevents the loss of these nucleotide sequences in absence of antibiotics in the growth medium.

The expression of heterologous nucleotide sequences encoded on plasmids depends on the number of plasmids present in the microorganism and thereby varies over time. Further, the expression of heterologous nucleotide sequences encoded on plasmids may lead to an unduly high expression, which may cause reduced activity of the gene products, for example, due to an impaired protein folding caused by the formation of inclusion bodies. Additionally, the high expression of heterologous nucleotide sequences encoded on plasmids requires a high consumption of cellular resources, which may also affect the protein activity and the proliferation of the microorganism. In contrast, the genomic integration of the heterologous nucleotide sequences ensures a constant copy number per cell over time and a moderate expression of the heterologous nucleotide sequences to enable the expression of natively folded, active proteins.

In a preferred embodiment of the method of producing vanillin according to the present invention, which can be combined with any of the preceding and subsequent embodiments, the method further comprises adding ferulic acid to a growth medium in which the microorganism is cultured. Particularly preferred are embodiments, in which the method further comprises producing 4-vinylguaiacol from ferulic acid as an intermediate.

In a particularly preferred embodiment of the method of producing vanillin according to the present invention, which can be combined with any of the preceding and subsequent embodiments, the ferulic acid is converted into vanillin with an efficiency of at least 95%, 97%, 98%, 99%, 99.1%, 99.2%, 99.3%, 99.4%, 99.5%, 99.6%, 99.7%, 99.8%, 99.9%, or 100%. In a particularly preferred embodiment, the ferulic acid is converted into vanillin with an efficiency of at least 99.4%. In a particularly preferred embodiment, the ferulic acid is converted into vanillin with an efficiency of at least 99.5%. In a particularly preferred embodiment, the ferulic acid is converted into vanillin with an efficiency of at least 99.6%. In a particularly preferred embodiment, the ferulic acid is converted into vanillin with an efficiency of at least 99.7%. In a particularly preferred embodiment, the ferulic acid is converted into vanillin with an efficiency of at least 99.8%. In a particularly preferred embodiment, the ferulic acid is converted into vanillin with an efficiency of at least 99.9%. In a particularly preferred embodiment, the ferulic acid is converted into vanillin with an efficiency of substantially 100%. In a particularly preferred embodiment, the ferulic acid is converted into vanillin with an efficiency of 100%.

In a preferred embodiment of the method of producing 4-vinylguaiacol or vanillin according to the present invention, which can be combined with any of the preceding and subsequent embodiments, the microorganism is a bacterium or a yeast. Particularly preferred are embodiments, in which the bacterium is *Escherichia coli.* Particularly preferred are the *E. coli* strains XL1-blue, TOP10, XL10 blue, DH5-alpha, JM109, C41, BL21gold (DE3) and W3110. In particularly preferred embodiments, the microorganism according to the present invention can be an *E. coli* strain TOP10. Particularly preferred is also the *E. coli* strain BL21gold (DE3). The method of producing 4-vinylguaiacol or vanillin is preferably performed by using dividing microorganism cells at conditions, which enable the growth of the cells.

In a preferred embodiment of the method of producing 4-vinylguaiacol or vanillin according to the present invention, which can be combined with any of the preceding and subsequent embodiments, the microorganism is cultivated in a growth medium in the absence of antibiotics. For example, the genomic integration of the heterologous nucleotide sequences that encode one or more of FADase, a first aromatic dioxygenase, a second aromatic dioxygenase and a vanillyl alcohol oxidase can prevent the loss of genetic information, for example through plasmid curation in the absence of antibiotics in the medium, and thereby enable cultivation of the microorganism in a growth medium in absence of antibiotics.

In some embodiments of the method of producing 4-vinylguaiacol or vanillin according to the present invention, which can be combined with any of the preceding and subsequent embodiments, the method further comprises extracting 4-vinylguaiacol or vanillin by using an organic solvent. The organic solvent can be methanol, ethanol, propanol, chloroform, acetone, cyclohexane, dimethyl sulfoxide, or diethyl ether. In preferred embodiments, the organic solvent is methanol. In particularly preferred embodiments, the purity of the extracted 4-vinylguaiacol or vanillin produced by a method according to the present invention is at least at 99%, 99.1%, 99.2%, 99.3%, 99.4%, 99.5%, 99.6%, 99.7%, 99.8%, 99.9%, or at 100%.

Particularly preferred are methods for producing 4-vinylguaiacol or vanillin with a purity of 100%. The term "purity" as used herein refers to the absence of by-products, such as other aromatic compounds. As described above, even minor traces of aromatic by-products can negatively affect the aroma of vanillin or 4-vinylguaiacol.

### Microorganism

A further aspect of the invention concerns a microorganism as used in the method of producing 4-vinylguiacol or in the method of producing vanillin as described herein. In particular the microorganism is able to produce vanillin or 4-vinylguaiacol from ferulic acid. The microorganism for the production of vanillin according to the present invention comprises heterologous nucleotide sequences integrated in the genome that encode a ferulic acid decarboxylase (FADase), and at least one of a first aromatic dioxygenase and a second aromatic dioxygenase.

In a preferred embodiment of the microorganism according to the present invention, the microorganism comprises the heterologous nucleotide sequences of FADase and TtAdo integrated in the genome; the microorganism comprises the heterologous nucleotide sequences of FADase and TtAdo2 integrated in the genome, or the microorganism contains the heterologous nucleotide sequences of FADase, TtAdo, and TtAdo2 integrated in the genome.

The microorganism for the production of 4-vinylguaiacol according to the present invention comprises a heterologous nucleotide sequence integrated into the genome that encodes ferulic acid decarboxylase (FADase). In a preferred embodiment, the microorganism is *Escherichia coli* which contains a heterologous nucleotide sequence that encodes ferulic acid decarboxylase (FADase) from *Enterobacter sp.* Px6-4, which catalyses the conversion of ferulic acid into 4-vinylguaiacol and has a nucleotide sequence according to SEQ ID NO: 1 or an amino acid sequence according to SEQ ID NO: 2, integrated into the genomic locus of *yahk.*

In preferred embodiments of the microorganism according to the present invention, which can be combined with any of the preceding and subsequent embodiments, the microorganism is a bacterium or a yeast. Particularly preferred are embodiments, in which the bacterium is *Escherichia coli.* Preferred are the *E. coli* strains XL1-blue, TOP10, XL10 blue, DH5-alpha, JM109, C41, BL21gold (DE3) and W3110. In particularly preferred embodiments, the microorganism according to the present invention can be an *E. coli* BL21gold (DE3). Alternatively, the microorganism according to the present invention can be an *E. coli* TOP10 strain.

In a particularly preferred embodiment of the microorganism according to the present invention, which can be combined with any of the preceding and subsequent embodiments, the microorganism for producing vanillin is an *Escherichia coli* BL21 (DE3) comprising the heterologous nucleotide sequences encoding for a ferulic acid decarboxylase (FADase), a first aromatic dioxygenase, a second aromatic dioxygenase, and a vanillyl alcohol oxidase integrated into the genomic locus of at least one of the endogenous alcohol dehydrogenases *yahk, yqhC, yqhD,* and/or *dkgA.*

A further aspect of the invention concerns the use of the microorganism according to the present invention for producing vanillin wherein the microorganism comprises heterologous nucleotide sequences integrated in the genome that encode a ferulic acid decarboxylase (FADase), and at least one of a first aromatic dioxygenase and a second aromatic dioxygenase.

Another aspect of the invention relates to the use of the microorganism according to the present invention for producing 4-vinylguaiacol wherein the microorganism comprises a heterologous nucleotide sequence integrated into the genome that encodes a ferulic acid decarboxylase (FADase). In some embodiments, the heterologous nucleotide sequence encoding FADase is integrated in the following genomic loci of alcohol dehydrogenases: *yahk, yqhC, yqhD, dkgA.*

### EXAMPLES

The invention is now described with reference to the following Examples. These Examples are provided for the purpose of illustration only, and the invention is not limited to these Examples but rather encompasses all variations that are evident as a result of the teachings provided herein.

### Example 1: Genome integration in E. coli

The nucleic acid sequences encoding for FADase, TtAdo, TtAdo2, and PsVAO were integrated into the genome of *Escherichia coli* BL21 (DE3) using the λ-phage-derived Red recombinases and homologous recombination, using approximately 500 bp long homology arms.

Briefly, the codon optimized version of the genes of FADase, TtAdo, TtAdo2 and PsVAO were synthetically synthesized and individually inserted into an integration plasmid upstream of a kanamycin resistance gene by using a PCR-based cloning method. The integration plasmid carried the p15A origin of replication, a kanamycin resistance cassette flanked by FRT and by homology arms. After cloning the genes to be integrated,the kanamycin resistance gene were flanked in the plasmid by homology arms of approximately 500 bp. The sequences of these homology arms were identical to the sequences flanking the integration site in the genome to enable homologous recombination.

The DNA fragment containing the homology arms, the genes to be integrated, and the kanamycin resistance gene was excised from the plasmid using restriction enzymes and electroporated into *E. coli* BL21 (DE3) expressing the λ-phage recombinases. Transformants were selected in rich media containing kanamycin (25 mg/l). The integration event was confirmed by colony PCR and sequencing. To perform a second round of integration, the kanamycin resistance cassette was removed, using the Flp/FRT system.

### Example 2: Generation of a 4-vinylguaiacol-producing E. coli.

The 4-vinylguaiacol-producing *E. coli* strain (herein referred to as *E. coli* strain #1) was generated by integrating the FADase gene from *Enterobacter sp.* Px6-4 into the gene *yahk* in wild type *E. coli* BL21 (DE3).

### Example 3: Generation and optimization of a vanillin-producing E. coli strain.

The *E. coli* strain for the production of vanillin was generated by carrying out several integration steps sequentially.

First, the nucleotide sequence encoding for the aromatic dioxygenase from *Myceliophthora thermophila* (TtAdo) was integrated into the *yqhC-dkgA* operon in the genome of *E. coli* strain #1 (the resulting strain is herein referred to as *E. coli* strain #2).

To improve vanillin production, a copy of the nucleotide sequence encoding for the aromatic dioxygenase from *Thermothielavioides terrestris* (TtAdo2) was integrated into the chromosome of *E. coli* strain #2, creating *E. coli* strain #3. To reduce the amount of vanillyl alcohol produced as an intermediate, a copy of the nucleotide sequence encoding for the vanillyl alcohol oxidase from *Penicillium simplicissimum* (PsVAO) was integrated into the genome of *E. coli* strain #3, resulting in *E. coli* strain #4, which can convert the vanillyl alcohol produced back to vanillin. Finally, to further increase vanillin production, a second copy of the TtAdo2 gene was integrated into *E. coli* strain #5, generating the final strain. The nucleic acid sequences encoding for TtAdo2 and PsVAO were integrated into non-essential genes, to avoid growth impairments of the *E. coli* strain.

As described above, the FADase gene was integrated into the *yahk* gene to prevent the expression of a functionally active alcohol dehydrogenase from this gene. Similarly, TtAdo was integrated into the *yqhC-dkgA* operon, so that the functionality of both genes (alcohol dehydrogenases) was disrupted. The Inventors surprisingly found that the inactivation of said alcohol dehydrogenases was not lethal for the *E. coli* strains. The inactivation of genes encoding for alcohol dehydrogenases reduces the conversion of vanillin to vanillyl alcohol and thereby improves the efficiency and purity of the method for producing vanillin from ferulic acid. In combination with the additional expression of PsVAO, which catalyses the conversion of vanillyl alcohol to vanillin, the method of the present invention allowed the production of highly pure vanillin in complete absence of vanillyl alcohol.

### Example 4: Vanillin production in non-dividing cells

As a standard growth medium, the synthetic media by *Riesenberg et al.* was used to generate the required cell biomass for the biotransformation experiments. The Riesenberg media consists of 1X Salt mix (13.3 g/l KH₂PO₄; 2,5 g/l (NH₄)₂HPO₄ and 1.95 g/l Citric acid), 1X trace elements (1000X: 0.65 mM Zn(CH₃COO)₂-2H₂O; 0.075 mM CuCl₂·2H₂O; 0.75 mM MnCl₂·4H₂O; 0.15 mM H₃BO₃; 0.42 mM Titriplex III (Na₂EDTA·2H₂O); 0.125 mM CoCl₂·6H₂O and 0.125 mM Na₂MoO₄·2H₂O) 4.9 mM MgSO₄, 0.2 mM FeCl₃, 0.13 mM Thiamine·HCl, 5 g/l yeast extract, 10 g/l glucose and 5 g/l arabinose. The media was supplemented with antibiotics if needed, Ampicillin (100 mg/l), Kanamycin (25 mg/l), and/or Chloramphenicol (25 mg/l).

For testing the production of vanillin in non-proliferating *E. coli* cells, 250 ml flasks containing 50 ml Riesenberg medium were inoculated with the vanillin-producing *E. coli* strain #5 starting at an OD600 of 0.05. These cultures were incubated at 28 °C, 200 rpm, for approximately 22 hours. After incubation, the cells were harvested by centrifugation and washed in buffer (Tris-HCl, pH 8.0) to remove any remaining media. Afterwards, the cells were resuspended in 13 ml buffer (Tris-HCl, pH 8.0), which enables evaluating the efficiency of the production of vanillin by non-proliferating cells. Commercial ferulic acid (5 mM), dissolved in dimethyl sulfoxide (DMSO), was added. 4 ml of non-dividing cells with ferulic acid as substrate were placed in screw-capped glass tubes in triplicates and incubated at 42 °C, 200 rpm for around 22 hours. After incubation, the products present in the non-dividing cell cultures were quantified.

### Example 5: 4-vinylguaiacol production in cultures

To test the production of 4-vinylguaiacol, 250 ml flasks containing 50 ml Riesenberg medium were inoculated with the 4-vinylguaiacol-producing *E. coli* strain #1 starting at an OD600 of 0.05. These cultures were incubated at 28 °C and 200 rpm for approximately 22 hours. After the incubation period, 5 mM ferulic acid, dissolved in DMSO, was added, and the cultures were incubated overnight at 28 °C, pH 7.0 and 200 rpm. After 24 hours incubation, the products present in the cultures (media and cells) were quantified.

### Example 6: Biotechnological production of Vanillin

A preculture of the *E. coli* strain #5 in Riesenberg media was grown in a shaking flask at 28 °C and 200 rpm for 20 - 24 hours. The fermenter containing 750 ml Riesenberg media supplemented with 15 g/l glucose was inoculated to a starting OD600 of 0.2 and it was allowed to grow for 17 - 22 hours at 28 °C and a pH value of 7. The pH value was regulated using 20% NH₃ and 50% H₃PO₄.

When the culture in the fermenter reached an OD600 of 20 - 25, the temperature was increased to 42 °C, the pH value was adjusted to 8, and ferulic acid dissolved in DMSO was added. The bioconversion was performed in the Riesenberg growth media under conditions that enabled bacterial growth. Further, the temperature of 42 °C led to the thermal inactivation of endogenous alcohol dehydrogenases. The biotransformation was completed after 48 hours. The products were extracted in methanol and analysed by HPLC (Figure 4A).

### Example 7: Biotechnological production of 4-vinylguaiacol

A preculture of the *E. coli* strain #1 in Riesenberg media was grown in a shaking flask at 28 °C and 200 rpm for 22 hours. The fermenter containing 750 ml Riesenberg media supplemented with 15 g/l glucose was inoculated to a starting OD600 of 0.2 and bacterial growth was enabled by incubation for 17 hours at 28 °C at a pH value of 7. The pH value was regulated using 20% NH₃ and 50% H₃PO₄.

When the culture in the fermenter reached an OD600 of 30, 5 mM ferulic acid dissolved in DMSO was added. The bioconversion was performed in Riesenberg growth media for 24 h at 28 °C and a pH value of 7, which enabled bacterial proliferation. The products were extracted with methanol and analysed by HPLC (Figure 4A).

### Example 8: Detection and quantification of 4-vinylguaiacol and vanillin

The yield of 4-vinylguaiacol or vanillin was analysed after the extraction of aromatic compounds by methanol. Therefore, a sample of 0.5 ml of the culture was mixed with 1 ml of methanol. After centrifugation, 10 µl of the supernatant were analysed by HPLC (Primaide, Hitachi) using a Purospher^{®} STAR RP18e 5µm (LiChroCART^{®} 125-4, Merck) column at a solvent flow rate of 1 ml/min at 40 °C.

The chromatography was performed with the following three-step gradient by using 1% formic acid as solvent A and acetonitrile: methanol 1:2 (v/v) as solvent B: 15% - 40% solvent B for 5 min, 40% - 70% solvent B for 3 min and 70% - 100% solvent B for 2 min. Substance detection was performed by using a photodiode array detector set at a wavelength of 280 nm. In addition, a spectrum from 200 nm-400 nm was recorded for each time point, which was used to confirm peak purity for each sample. As analytical standards, commercially available compounds were used: ferulic acid (99% purity, Merck), vanillin (Merck), vanillic acid (98%, AlfaAesar), vanillyl alcohol (98%, Sigma), 4-vinylguaiacol (98%, Sigma). The HPLC chromatograms of the extracted products of the production of 4-vinylguaiacol and vanillin are depicted in Figure 5A and Figure 5B, respectively.

For determining the amount of 4-vinylguaiacol or vanillin, the area of the corresponding peaks in the chromatogram was calculated, which is directly proportional to the amount of substance. For the generation of a reference curve, increasing amounts of pure reference substances were analysed in this manner. Therefore, the HPLC chromatograms of 5 different concentration of the pure reference standards (0.005 mg to 0.06 mg), dissolved in methanol, were measured in triplicates of 10 µl each. The peak areas of the reference standards were plotted against the corresponding concentrations of the reference standards for calculating the reference curve by linear regression.

By using this reference curve, the mass concentrations of the extracted products from the bioconversion were calculated from the peak areas based on the reference curve. The mass concentrations of these products were used to calculate the molar amount by dividing it by the corresponding molecular weight. The efficiency of production was calculated by taking the total mass concentration of all products present in the sample as 100%.

In summary, the present invention provides a novel and inventive biotechnological method for the production of vanillin and 4-vinylguaiacol from ferulic acid with improved efficiency of substantially 100%. The present invention provides a sustainable, cost-effective, and scalable way to meet the increasing demand for vanillin by various industries. The invention opens new possibilities for the production of natural flavours and fragrances with improved purity and efficiency.

### BIBLIOGRAPHY

Armanino, N., Charpentier, J., Flachsmann, F., Goeke, A., Liniger, M., Kraft, P. (2020). What's Hot, What's Not: The Trends of the Past 20 Years in the Chemistry of Odourants. Angewandte Chemie Internationale Edition, 59(38):16310-16344.
Banerjee, G., & Chattopadhyay, P. (2019). Vanillin biotechnology: the perspectives and future. Journal of the Science of Food and Agriculture (Vol. 99, Issue 2, pp. 499-506).
Baqueiro-Peña, I., Rodriguez-Serrano, G., González-Zamora, E., Augur, C., Loera, O., Saucedo-Castañeda, G. (2010). Biotransformation of ferulic acid to 4-vinylguaiacol by a wild and a diploid strain of Aspergillus niger. Bioresource Technology (Vol. 101, Issue 12, pp. 4721-4724).
di Gioia, D., Luziatelli, F., Negroni, A., Ficca, A. G., Fava, F., & Ruzzi, M. (2011). Metabolic engineering of Pseudomonas fluorescens for the production of vanillin from ferulic acid. Journal of Biotechnology, 156(4), 309-316.
Fache, M., Boutevin, B., & Caillol, S. (2016). Vanillin Production from Lignin and Its Use as a Renewable Chemical. ACS Sustainable Chemistry and Engineering, 4(1), 35-46.
Fleige, C., Meyer, F., & Steinbüchel, A. (2016). Metabolic engineering of the actinomycete Amycolatopsis sp. strain ATCC 39116 towards enhanced production of natural vanillin. Applied and Environmental Microbiology, 82(11), 3410-3419.
Furuya, T., Miura, M., & Kino, K. (2014). A coenzyme-independent decarboxylase/oxygenase cascade for the efficient synthesis of vanillin. Chembiochem : A European Journal of Chemical Biology, 15(15), 2248-2254.
Hua, D., Ma, C., Song, L., Lin, S., Zhang, Z., Deng, Z., & Xu, P. (2007). Enhanced vanillin production from ferulic acid using adsorbent resin. Applied Microbiology and Biotechnology, 74(4), 783-790.
Lee, E. G., Yoon, S. H., Das, A., Lee, S. H., Li, C., Kim, J. Y., Choi, M. S., Oh, D. K., & Kim, S. W. (2009). Directing vanillin production from ferulic acid by increased acetyl-CoA consumption in recombinant Escherichia coli. Biotechnology and Bioengineering, 102(1), 200-208.
Hansen, E. H., Moller, B. L., Kock, G. R., Bünner, C. M., Kristensen, C., Jensen, O. R., Okkels, F. T., Olsen, C. E., Motawia, M. S., & Hansen, J. (2009). De novo biosynthesis of Vanillin in fission yeast (Schizosaccharomyces pombe) and baker's yeast (Saccharomyces cerevisiae). Applied and Environmental Microbiology, 75(9), 2765-2774.
Havkin-Frenkel, D. (Daphna), Belanger, F. C., & Blackwell Publishing Ltd. (2008). Biotechnology in flavour production. Blackwell Publishing.
Knapp, W., Schmudermayer, A., Seiberl, W., Sigl, K., & Eder, R. (2010). Sensorische Beschreibung und Ermittlung der Wahrnehmungsschwelle von flüchtigen Phenolen und Isovaleriansäure. Mitteilungen Klosterneuburg, 60:268-277.
Li, L., Long, L., & Ding, S. (2019). Bioproduction of high-concentration 4-vinylguaiacol using whole-cell catalysis harboring an organic solvent-tolerant phenolic acid decarboxylase from Bacillus atrophaeus. Frontiers in Microbiology, 10(AUG).
Liu, Y., Sun, L., Huo, Y. X., & Guo, S. (2023). Strategies for improving the production of bio-based vanillin. In Microbial Cell Factories (Vol. 22, Issue 1). BioMed Central Ltd.
Ma, X. K., & Daugulis, A. J. (2014). Effect of bioconversion conditions on vanillin production by Amycolatopsis sp. ATCC 39116 through an analysis of competing by-product formation. Bioprocess and Biosystems Engineering, 37(5), 891-899.
Ma, Q., Liu, L., Zhao, S., Huang, Z., Li, C., Jiang, S., Li, Q., & Gu, P. (2022). Biosynthesis of vanillin by different microorganisms: a review. In World Journal of Microbiology and Biotechnology (Vol. 38, Issue 3). Springer Science and Business Media B.V.
Ni, J., Wu, Y. T., Tao, F., Peng, Y., & Xu, P. (2018). A Coenzyme-Free Biocatalyst for the Value-Added Utilization of Lignin-Derived Aromatics. Journal of the American Chemical Society, 140(47), 16001-16005.
Overhage, J., Priefert, H., Rabenhorst, J., & Steinbu6, A. (n.d.). Biotransformation of eugenol to vanillin by a mutant of Pseudomonas sp. strain HR199 constructed by disruption of the vanillin dehydrogenase (vdh) gene.
Pérez-Rodríguez, N., Pinheiro de Souza Oliveira, R., Torrado Agrasar, A. M., & Domínguez, J. M. (2016). Ferulic acid transformation into the main vanilla aroma compounds by Amycolatopsis sp. ATCC 39116. Applied Microbiology and Biotechnology, 100(4), 1677-1689.
Tinikul, R., Chenprakhon, P., Maenpuen, S., Chaiyen, P. (2017). Biotransformation of Plant Derived Phenolic Acids. Biotechnology Journal, (Vol 13, Issue 6 pp.).
Serra, S., Fuganti, C., & Brenna, E. (2005). Biocatalytic preparation of natural flavours and fragrances. In Trends in Biotechnology (Vol. 23, Issue 4, pp. 193-198).
Sun, LH., Lv, SW., Yu, F., Li, SN., He, LY. (2018). Biosynthesis of 4-vinylguaiacol from crude ferulic acid by Bacillus licheniformis DLF-17056. Journal of Biotechnology (Vol. 281, pp. 144-149).
Valério, R., Bernardino, A. R. S., Torres, C. A. V., Brazinha, C., Tavares, M. L., Crespo, J. G., & Reis, M. A. M. (2021). Feeding strategies to optimize vanillin production by Amycolatopsis sp. ATCC 39116. Bioprocess and Biosystems Engineering, 44(4), 737-747.
Wang, Q., Wu, X., Lu, X. Ma, B., Xu, Y. (2021). Efficient Biosynthesis of Vanillin from Isoeugenol by Recombinant Isoeugenol Monooxygenase from Pseudomonas nitroreducens Jin1. Appl Biochem Biotechnol 193, 1116-1128.
Zhao, L., Xie, Y., Chen, L., Xu, X., Zhao, C. X., & Cheng, F. (2018). Efficient biotransformation of isoeugenol to vanillin in recombinant strains of Escherichia coli by using engineered isoeugenol monooxygenase and sol-gel chitosan membrane. Process Biochemistry, 71, 76-81.
Zhao, M., Hong, X., Abdullah, Yao, R., & Xiao, Y. (2021). Rapid biosynthesis of phenolic glycosides and their derivatives from biomass-derived hydroxycinnamates. Green Chemistry, 23(2), 838-847.

## Claims

1. A method of producing vanillin comprising culturing a microorganism that comprises heterologous nucleotide sequences integrated in the genome that encode
a. a ferulic acid decarboxylase (FADase), and at least one of
b. a first aromatic dioxygenase and
c. a second aromatic dioxygenase.

2. The method according to claim 1, wherein the nucleotide sequence that encodes the first aromatic dioxygenase has at least 70% or at least 80%, 85%, 90%, 91%, 92%, 93%, 94%, 95%, 96%, 97%, 98%, 99% or 100% sequence identity with the sequence of the aromatic dioxygenase from *Myceliophthora thermophila* according to SEQ ID NO. 3 or wherein the aromatic dioxygenase has at least 80%, 85%, 90%, 91%, 92%, 93%, 94%, 95%, 96%, 97%, 98%, 99% or 100% sequence identity with the amino acid sequence of aromatic dioxygenase from *Myceliophthora thermophila* according to SEQ ID NO. 4.

3. The method according to any one of claims 1 or 2, wherein the nucleotide sequence that encodes the second aromatic dioxygenase has at least 70% or at least 80%, 85%, 90%, 91%, 92%, 93%, 94%, 95%, 96%, 97%, 98%, 99% or 100% sequence identity with the sequence of the aromatic dioxygenase from *Thermothielavioides terrestris* according to SEQ ID NO. 5 or wherein the aromatic dioxygenase has at least 80%, 85%, 90%, 91%, 92%, 93%, 94%, 95%, 96%, 97%, 98%, 99% or 100% sequence identity with the amino acid sequence of aromatic dioxygenase from *Thermothielavioides terrestris* according to SEQ ID NO. 6.

4. The method according to any one of claims 1 to 3, wherein the microorganism further comprises a heterologous nucleotide sequence that encodes for a vanillyl alcohol oxidase.

5. The method according to claim 4, wherein the heterologous nucleotide sequence that encodes the vanillyl alcohol oxidase has at least 70% or at least 80%, 85%, 90%, 91%, 92%, 93%, 94%, 95%, 96%, 97%, 98%, 99% or 100% sequence identity with the sequence of the vanillyl alcohol oxidase from *Penicillium simplicissimum* according to SEQ ID NO. 7 or wherein the vanillyl alcohol oxidase has at least 80%, 85%, 90%, 91%, 92%, 93%, 94%, 95%, 96%, 97%, 98%, 99% or 100% sequence identity with the amino acid sequence of the vanillyl alcohol oxidase from *Penicillium simplicissimum* according to SEQ ID No. 8, preferably wherein the heterologous nucleotide sequence that encodes the vanillyl alcohol oxidase is integrated in the genome.

6. The method according to any one of claims 1 to 5, wherein the method further comprises adding ferulic acid to a growth medium in which the microorganism is cultured and producing 4-vinylguaiacol from ferulic acid as an intermediate.

7. The method according to any one of claims 1 to 6, wherein the microorganism has a reduced expression of *yahK, yqhC, yqhD,* and/or *dkgA* compared to the expression of the respective gene in the wild type microorganism.

8. The method according to claim 7, wherein the reduced expression results from the integration of genetic elements into the genes of *yahK, yqhC, yqhD,* and/or *dkgA.*

9. The method according to any one of claims 1 to 8, wherein ferulic acid is converted into vanillin with an efficiency of at least 95%, 97%, 98%, 99%, 99.1%, 99.2%, 99.3%, 99.4%, 99.5%, 99.6%, 99.7%, 99.8%, 99.9%, or 100%.

10. A method of producing 4-vinylguaiacol comprising culturing a microorganism that comprises a heterologous nucleotide sequence integrated in the genome that encodes ferulic acid decarboxylase (FADase).

11. The method according to any one of claims 1 to 10, wherein the nucleotide sequence that encodes the ferulic acid decarboxylase (FADase) has at least 70% or at least 80%, 85%, 90%, 91%, 92%, 93%, 94%, 95%, 96%, 97%, 98%, 99% or 100% sequence identity with the sequence of ferulic acid decarboxylase from *Enterobacter sp.* Px6-4 according to SEQ ID NO. 1 or wherein the ferulic acid decarboxylase (FADase) has at least 80%, 85%, 90%, 91%, 92%, 93%, 94%, 95%, 96%, 97%, 98%, 99% or 100% sequence identity with the amino acid sequence of ferulic acid decarboxylase from *Enterobacter sp.* Px6-4 according to SEQ ID NO. 2.

12. The method according to any one of claims 1 to 11, wherein the microorganism is a bacterium or a yeast, preferably wherein the bacterium is Escherichia coli.

13. The method of claims 1 to 12, wherein the microorganism is cultivated in a growth medium in the absence of antibiotics.

14. A microorganism as defined in any one of claims 1 to 13.

15. Use of the microorganism of claim 14 for producing vanillin or 4-vinylguaiacol.
